(19) Europäisches Patentamt
European Patent Office
Office européen des brevets

(11) **EP 2 562 541 A1**

(12) **EUROPÄISCHE PATENTANMELDUNG**

(43) Veröffentlichungstag:
27.02.2013 Patentblatt 2013/09

(51) Int Cl.:
*G01N 33/00* (2006.01)　　　*G01F 1/74* (2006.01)

(21) Anmeldenummer: 11178490.6

(22) Anmeldetag: 23.08.2011

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME

(71) Anmelder: **Siemens Aktiengesellschaft**
**80333 München (DE)**

(72) Erfinder:
• **Braun, Gilbert**
**52134 Herzogenrath (DE)**
• **Kury-Thien, Peter**
**45127 Essen (DE)**

(54) **Hochgenaue Bestimmung des Masseanteils einer Komponente in einem Mehrkomponenten-Fluid**

(57)　Zur Bestimmung eines Masseanteils einer ersten Komponente (W) eines Mehrkomponenten-Fluids (F) ist vorgesehen, die erste Komponente (W) in einem Trennschritt zumindest teilweise aus dem Mehrkomponenten-Fluid (F) abzutrennen, mindestens zwei Referenz-Durchflussraten ($M_1$,$M_2$) zu bestimmen, die gewählt sind aus
- einer Durchflussrate ($m_1$) des dem Trennschritt zugeführten Mehrkomponenten-Fluids (F),
- einer Durchflussrate ($m_2$) eines aus der Abtrennung der ersten Komponente (W) resultierenden Rest-Fluids (T),

und
- einer Durchflussrate ($m_3$) eines in dem Trennschritt anfallenden Trenn-Fluids (K), und
aus den gewählten Referenz-Durchflussraten ($M_1$,$M_2$) den Masseanteil ($X_W$) der ersten Komponente (W) unter Berücksichtigung eines nicht abgetrennten Restanteils ($m_{W2}$) der ersten Komponente (W) in dem Rest-Fluid (T) zu bestimmen. Erfindungsgemäß wird weiterhin ein Fremdanteil ($m_{D3}$) einer in dem Trennschritt mitabgeteilten weiteren Komponente (D) des Mehrkomponenten-Fluids (F) berücksichtigt.

## FIG 2

EP 2 562 541 A1

**Beschreibung**

Die Erfindung bezieht sich auf ein Verfahren zur Bestimmung eines Masseanteils einer Komponente eines Mehrkomponenten-Fluids. Der Begriff "Fluid" bezeichnet allgemein ein Gas, eine Flüssigkeit oder eine aus gasförmigen, flüssigen und/oder festen Anteilen bestehende, fließfähige Phasenmischung. Der Begriff "Komponente" bezeichnet hierin allgemein einen abtrennbaren Anteil des Mehrkomponenten-Fluids mit bestimmten chemischen und/oder physikalischen Eigenschaften, anhand derer diese Komponente von mindestens einer weiteren Komponente des Mehrkomponenten-Fluids unterscheidbar ist. Die Komponenten des Mehrkomponenten-Fluids werden hierbei insbesondere durch verschiedene chemische Stoffe gebildet. Grundsätzlich kann eine Komponente des Mehrkomponenten-Fluids aber auch bereits selbst aus einer Mischung verschiedener chemischer Stoffe gebildet sein. Ebenso können verschiedene Phasen (Aggregatzustände) desselben chemischen Stoffes oder derselben chemischen Stoffmischung verschiedene Komponenten des Mehrkomponenten-Fluids bilden. Diejenige Komponente, deren Masseanteil verfahrensgemäß bestimmt werden soll, ist nachfolgend - zur begrifflichen Unterscheidung von weiteren Komponenten des Mehrkomponenten-Fluids - auch als "erste Komponente" bezeichnet.

Bei dem Mehrkomponenten-Fluid handelt es sich insbesondere um ein Synthesegas, wie es zum Beispiel als Brenngas zur Verbrennung in Kraftwerks-Gasturbinen eingesetzt wird. Ein solches Synthesegas besteht üblicherweise aus Wasserstoff ($H_2$), Kohlenmonoxid (CO), Kohlendioxid ($CO_2$), Stickstoff ($N_2$) sowie Wasser ($H_2O$). Der Wassergehalt wird üblicherweise zum Beispiel mittels Dampfzumischung variiert, um die Reaktivität des Brenngases so einzustellen, dass geforderte Emissionsgrenzen (zum Beispiel hinsichtlich des Stickoxid ($NO_x$)-Ausstoßes) eingehalten werden. Der Wassergehalt eines typischen Synthesegases kann 50% erreichen oder übersteigen.

Bei der Verbrennung eines Synthesegases in einer Kraftwerks-Gasturbine ist eine Echtzeit-Gasanalyse wünschenswert, da die Gaszusammensetzung und damit die für die Verbrennung in der Gasturbine wichtigen Größen (zum Beispiel Wobbe-Index und Reaktivität) zeitlichen Schwankungen unterliegen, die aus dem Vergasungsprozess herrühren und zum Beispiel durch wechselnde Prozessbedingungen oder Ausgangsmaterialien verursacht sein können.

Aufgrund der hohen Brenngastemperaturen im Bereich von etwa 200° Celsius und des hohen Wasseranteils üblicher Synthesegase können gängige Methoden zur Gasanalyse, insbesondere Infrarotabsorptionsmessung und Gas-Chromatographie nicht direkt auf das Brenngas angewendet werden. Vielmehr ist vor der Anwendung einer solchen Gasanalyse in der Regel eine Trocknung und Kühlung des Brenngases erforderlich. Nachteiligerweise führt dieser Kühl- und Trocknungsprozess aber bereits zu einer erheblichen Verfälschung der ursprünglichen Gaszusammensetzung, zumal ein erheblicher Teil des ursprünglich im Synthesegas enthaltenen Wassers entfernt wird.

Aus DE 44 33 451 A1 ist ein Verfahren zur Bestimmung des Wassergehaltes in einem Gas bekannt, bei dem der Volumen- oder Massenstrom des zu analysierenden Gases durch Messung des über einem Strömungswiderstand abfallenden Differenzdrucks bestimmt wird. Anschließend an diese Messung wird das in dem Gas enthaltene Wasser in einem Trennschritt zumindest großenteils durch Kondensation entfernt. Im Anschluss an den Trennschritt wird der Massen- oder Volumenstrom des aus dem Trennschritt resultierenden, getrockneten Gases bestimmt. Diese Messung erfolgt wiederum indirekt über den über einem Strömungswiderstand abfallenden Differenzdruck. Aus der Differenz der gemessenen Massen- oder Volumenströme wird hierbei auf den Masseanteil des ursprünglich in dem Gas enthaltenen Wassers geschlossen. Hierbei wird die Restfeuchte des getrockneten Gases als Korrekturfaktor berücksichtigt. Das getrocknete und gekühlte Gas kann anschließend einem Gasanalysesystem zugeführt werden.

Der Erfindung liegt die Aufgabe zugrunde, ein einfach umsetzbares, gleichzeitig aber hochgenaues Verfahren zur Bestimmung des Masseanteils einer Komponente eines Mehrkomponenten-Fluids anzugeben. Das Verfahren soll dabei insbesondere eine hochgenaue Bestimmung des Wasseranteils eines Synthesegases unter den in einer Kraftwerks-Gasturbine üblicherweise vorherrschenden Bedingungen ermöglichen. Der Erfindung liegt weiterhin die Aufgabe zugrunde, eine zur Durchführung des Verfahrens besonders geeignete Einrichtung anzugeben.

Bezüglich des Verfahrens wird die Aufgabe erfindungsgemäß gelöst durch die Merkmale des Anspruchs 1. Bezüglich der zugehörigen Einrichtung wird die Aufgabe erfindungsgemäß gelöst durch die Merkmale des Anspruchs 11. Vorteilhafte Ausgestaltungen und Weiterentwicklungen der Erfindung sind in den Unteransprüchen niedergelegt.

Bei dem erfindungsgemäßen Verfahren wird zur Bestimmung eines Masseanteils einer ersten Komponente eines Mehrkomponenten-Fluids der eingangs definierten Art die erste Komponente in einem Trennschritt zumindest teilweise aus dem Mehrkomponenten-Fluid abgetrennt. Als "Mehrkomponenten-Fluid" wird zur begrifflichen Trennung im Folgenden nur das dem Trennschritt zugeführte Fluid bezeichnet, das die erste Komponente noch in dem vollen, zu bestimmenden Masseanteil enthält. Das aus dem Trennschritt unter vollständiger oder teilweiser Entfernung der ersten Komponente resultierende Fluid ist dagegen nachfolgend als "Rest-Fluid" bezeichnet. Das im Zuge des Trennschrittes anfallende, die abgetrennte erste Komponente enthaltene Fluid ist nachfolgend als "Trenn-Fluid" bezeichnet.

Sowohl dem (dem Trennschritt zugeführten) Mehrkomponenten-Fluid, als auch dem (aus dem Trennschritt resultierenden) Rest-Fluid, als auch dem (im Trennschritt anfallenden) Trenn-Fluid ist jeweils eine Durchflussrate zugeordnet, die jeweils beispielsweise in Einheiten eines Massen- oder Volumenflusses angegeben werden kann. Im Zuge des erfindungsgemäßen Verfahrens werden mindestens zwei dieser drei Flussraten als Referenz-Flussraten bestimmt.

In einem Auswertungsschritt des Verfahrens wird aus diesen Referenz-Flussraten der Masseanteil der ersten Komponente bestimmt.

Um eine Verfälschung des berechneten Masseanteils bei einer nicht-idealen Abtrennung der ersten Komponente zu vermeiden, wird bei der Berechnung dieses Masseanteils einerseits ein (nicht abgetrennter) Restanteil der ersten Komponente in dem Rest-Fluid berücksichtigt. Erfindungsgemäß wird zusätzlich bei der Berechnung des Masseanteils der ersten Komponente ein Anteil mindestens einer weiteren Komponente berücksichtigt, der in dem Trennschritt mitabgeteilt wird, und der somit als "Fremd-Anteil" in dem Trenn-Fluid enthalten ist.

Bei dem Mehrkomponenten-Fluid handelt es sich insbesondere um ein Synthesegas der vorstehend beschriebenen Art. Bei der ersten Komponente, deren Masseanteil verfahrensgemäß zu bestimmen ist, handelt es sich insbesondere um Wasser. In diesem Anwendungsfall wird verfahrensgemäß insbesondere der Anteil des mit dem Wasser stets in gewissem Umfang mit abgetrennten Kohlendioxids als Fremdanteil berücksichtigt. Grundsätzlich kann das erfindungsgemäße Verfahren aber auch auf andere Mehrkomponenten-Fluide angewendet werden, beispielsweise auf die Bestimmung des Masseanteils von Sand (als erste Komponente) in einem Wasser-Sandgemisch. In diesem Anwendungsfall wird verfahrensgemäß der Anteil des zusammen mit dem Sand abgetrennten Wassers als Fremdanteil berücksichtigt.

Zur Erzielung einer besonders hohen Messgenauigkeit werden die in die Berechnung eingehenden Referenz-Durchflussraten vorzugsweise nicht indirekt, sondern durch direkte Messung des Massenflusses des Mehrkomponenten-Fluids, des Rest-Fluids bzw. des Trenn-Fluids bestimmt. Für die direkte Massenflussmessung wird hierbei vorzugsweise jeweils ein Coriolis-Massenflussmesser eingesetzt.

In einer zweckmäßigen Ausführungsform des Verfahrens wird der Druck des Mehrkomponenten-Fluids auf einen vorgegebenen Sollwert geregelt und somit - im Rahmen der Toleranz der Regelung - konstant gehalten. Diese Maßnahme beruht auf der Erkenntnis, dass Druckschwankungen stets zu einer Störung der Durchflussraten- bzw. Massenbilanz der in den Trennschritt eingehenden bzw. aus dem Trennschritt hervorgehenden Fluidströme führen. Konkret summieren sich - anders als im stationären Fall - während einer Druckschwankung die Durchflussraten des Mehrkomponenten-Fluids, des Rest-Fluids und des Trenn-Fluids regelmäßig nicht zu Null, zumal das diese Fluide führende Leitungssystem eine - druckabhängig variierende - Speicherkapazität aufweist. So wird in der Regel bei steigendem Druck eine zunehmende Fluidmenge im Leitungssystem gespeichert, was regelmäßig dazu führt, dass die Durchflussrate des Mehrkomponenten-Fluids die Summe der Durchflussraten des Trenn-Fluids und des Rest-Fluids vorübergehend überschreitet. Eine entgegengesetzte Durchflussratendifferenz tritt bei fallendem Fluiddruck auf. Erkanntermaßen führen diese Effekte zu teils erheblichen Fehlern bei der Bestimmung des Masseanteils der ersten Komponente, die durch die Druckregelung auf besonders einfache und effektive Weise vermieden werden.

Eine weitere Verbesserung der Messgenauigkeit wird in einer bevorzugten Verfahrensvariante dadurch erzielt, dass regelungsbedingte Druckschwankungen (so genannte Regelschwingungen) durch ein dem Druckregler nachgeschaltetes Dämpfungsglied eliminiert oder zumindest reduziert werden. Als Dämpfungsglied wird hierbei in besonders einfacher und vorteilhafter Ausführung ein Filter verwendet. Im Anwendungsfall auf Synthesegase wird hierbei insbesondere ein grobmaschiger Gasfilter zum Beispiel aus Sintermaterial oder Keramik, mit einer Porengröße zwischen 200 und 500 $\mu$m verwendet.

Sofern es sich bei dem Mehrkomponenten-Fluid um einen Gasstrom handelt, erfolgt die Abtrennung der ersten Komponente vorzugsweise durch Kondensation in einem Kondensator (auch als Gas-Kühler oder Kühlfalle bezeichnet). Die Verwendung eines solchen Kondensators ist insbesondere für die Anwendung des Verfahrens auf ein Synthesegas für eine Gasturbine sinnvoll, zumal das Synthesegas durch den Kondensator gleichzeitig auf eine für die weitere Gasanalyse geeignete Temperatur abgekühlt wird.

Der Restanteil der ersten Komponente in dem Rest-Fluid kann im Rahmen der Erfindung grundsätzlich durch Messung bestimmt oder anhand von Erfahrungswerten abgeschätzt werden. Ersteres ist allerdings regelmäßig mit vergleichsweise großem Aufwand, letzteres mit vergleichsweise großer Ungenauigkeit verbunden. Sofern die Abtrennung der ersten Komponente durch Kondensation erfolgt, hat es sich dagegen als sowohl einfach realisierbar als auch präzise herausgestellt, den Restanteil aus der Kondensationskurve der ersten Komponente zu bestimmen. Hierbei wird zweckmäßigerweise der (aus der Kodensationskurve ablesbare) Sättigungsdampfdruck der ersten Komponente bei der im Kondensator herrschenden Temperatur (Kondensatortemperatur) herangezogen.

Der Fremdanteil der weiteren Komponente in dem Trenn-Fluid wird dagegen vorzugsweise anhand der Löslichkeit der weiteren Komponente in der ersten Komponente bestimmt. Dabei wird zweckmäßigerweise wiederum der Wert der Löslichkeit bei der Kondensatortemperatur herangezogen.

In einer zweckmäßigen Ausgestaltung des Verfahrens wird der Masseanteil der ersten Komponente nach der Gleichung

$$x_W = \frac{1}{1+\beta} + \left(1 - \frac{1}{1+\beta} - \frac{1}{1+\alpha}\right)\frac{M_2}{M_1}$$

bestimmt. In dieser Gleichung stehen

- $X_W$      für den verfahrensgemäß zu bestimmenden Masseanteil der ersten Komponente,
- $T_K$      für die Kondensatortemperatur,
- $\alpha = \alpha(T_K)$      für den dem Sättigungsdampfdruck bei der Kondensatortemperatur entsprechende Massedichteanteil der ersten Komponente im Restgas,
- $\beta = \beta(T_K)$      für die Löslichkeit der weiteren Komponente in der ersten Komponente bei der Kondensatortemperatur,
- $M_1$      für die als erste Referenz-Durchflussrate gewählte Durchflussrate des dem Trennschritt zugeführten Mehr-komponenten-Fluids, und
- $M_2$      für die als zweite Referenz-Durchflussrate gewählte Durchflussrate des durch die Abtrennung der ersten Komponente aus dem Trennschritt resultierenden Rest-Fluids.

In einer vorteilhaften Erfindungsvariante wird das freie Gasvolumen in dem gegebenenfalls zur Abtrennung der ersten Komponente herangezogenen Kondensator durch geregelten Abzug des Trenn-Fluids aus dem Kondensator konstant gehalten. Auch mit dieser Maßnahme wird - ähnlich wie mit der zuvor beschriebenen Druckregelung - die Speicherkapazität des das Fluid führenden Leitungssystems zumindest näherungsweise konstant gehalten, was sich positiv auf die erzielbare Messgenauigkeit auswirkt.

Die erfindungsgemäße Einrichtung umfasst in zweckmäßiger Ausbildung eine Trenneinheit zur zumindest teilweisen Abtrennung der ersten Komponente aus dem Mehrkomponenten-Fluid. Die Einrichtung umfasst weiterhin eine erste Durchflussraten-Messeinheit sowie eine zweite Durchflussraten-Messeinheit zur Messung jeweils einer der mindestens zwei Referenz-Durchflussraten, wobei es sich bei diesen Durchflussraten-Messeinheiten vorzugsweise um Coriolis-Massenflussmessgeräte handelt.

Die erfindungsgemäße Einrichtung umfasst schließlich eine Auswerteeinheit, die zur Ausführung des vorstehend beschriebenen Verfahrens in einer seiner Varianten ausgebildet ist. Konkret ist die Auswerteeinheit dazu eingerichtet, aus den Referenz-Durchflussraten den Masseanteil der ersten Komponente zu bestimmen, wobei sie erfindungsgemäß sowohl den nicht abgetrennten Restanteil der ersten Komponente in dem Rest-Fluid als auch den Fremdanteil der mitabgeteilten weiteren Komponente in dem Trenn-Fluid berücksichtigt.

Bei der Auswerteeinheit handelt es sich in bevorzugter Ausbildung der Einrichtung insbesondere um einen Rechner (Computer), in dem die zur automatischen Durchführung des Verfahrens erforderlichen Anweisungen in Form eines Steuerprogramms softwaretechnisch implementiert sind.

Zur Vergleichmäßigung des Fluiddrucks und somit zur Verbesserung der Messgenauigkeit ist der Trenneinheit vorzugsweise ein Druckregler vorgeschaltet. Zur Reduzierung von regelungsbedingten Druckschwankungen ist dem Druckregler und der Trenneinheit vorzugsweise ein Filter, insbesondere ein Filter der vorstehend beschriebenen Art, zwischengeschaltet. In Anwendung der Einrichtung auf die Analyse von Gasströmen ist die Trenneinheit vorzugsweise als Kondensator (auch: Gas-Kühler oder Kühlfalle) ausgebildet. Dem Kondensator ist hierbei vorzugsweise ein Füllstandsregler beigeordnet, der - im Rahmen vorgegebener Regelungstoleranzen - die Konstanthaltung des freien Gasvolumens im Kondensator durch geregelten Abzug des Trenn-Fluids bewirkt.

Nachfolgend werden Ausführungsbeispiele der Erfindung anhand einer Zeichnung näher erläutert. Darin zeigen:

FIG 1      grob schematisch vereinfacht ein Gasturbinen-Kraftwerk mit einer Gasturbine und einem Hauptgasstrang zur Zuführung eines Synthesegases zu der Gasturbine, sowie einen von dem Hauptgasstrang abzweigenden Probegasstrang zur Echtzeit-Analyse des Synthe- segases, wobei in dem Probegasstrang eine Einrichtung zur Bestimmung des Masseanteils des in dem Synthesegas enthaltenen Wassers angeordnet ist, und

FIG 2      in schematischer Darstellung die in dem Probegasstrang fließenden Masseströme.

Einander entsprechende Teile und Größen sind in allen Figuren stets mit gleichem Bezugszeichen versehen.

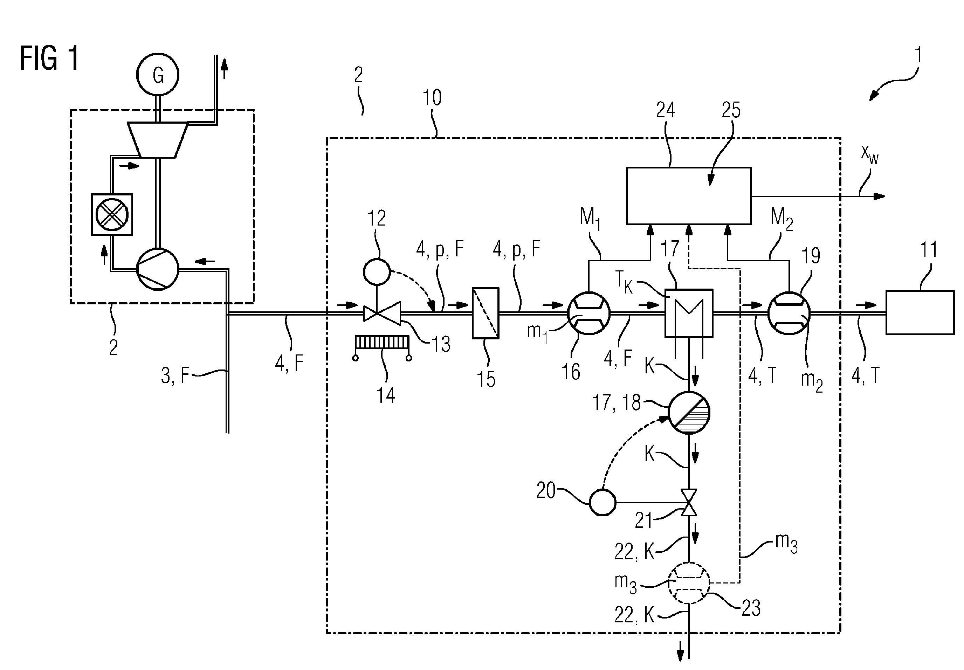

In FIG 1 ist grob schematisch vereinfacht ein (Gasturbinen-) Kraftwerk 1 dargestellt. Den zentralen Bestandteil des Kraftwerks 1 bildet mindestens eine Gasturbine 2, der über einen (Haupt-)Gasstrang 3 ein verdichtetes Synthesegas zur Verbrennung zugeführt ist. Dieses (stark wasserhaltige) Synthesegas ist nachfolgend als Feuchtgas F bezeichnet. Bei dem Feuchtgas F handelt es sich um ein Mehrkomponenten-Fluid im Sinne der vorstehenden Definition, das aus mehreren gasförmigen Komponenten, nämlich aus Wasser ($H_2O$) sowie aus Kohlenmonoxid (CO), Kohlendioxid ($CO_2$), Wasserstoff ($H_2$), Stickstoff ($N_2$) sowie gegebenenfalls Spuren weiterer Stoffe zusammengesetzt ist. Das Feuchtgas F wird hierbei insbesondere in einem Vergasungsprozess z.B. aus Biomasse, Kohle, Teer oder Asphalt durch fette (unterstöchiometrische) teilweise Verbrennung mit Sauerstoff ("ogygen blown") oder Luft ("air blown") unter Zugabe von Wasser hergestellt.

Das Feuchtgas F hat insbesondere eine Temperatur von ca. 200°C, einen Druck von ca. 35 bar. Es hat einen (schwankenden) Masseanteil an Wasser, der typischerweise in der Größenordnung von etwa 50% liegt.

Um Schwankungen in der Zusammensetzung des Feuchtgases F entgegenzuwirken, und somit die Verbrennung zu optimieren, wird das Feuchtgas F in Echtzeit analysiert. Hierzu ist von dem Hauptgasstrang 3 ein Probegasstrang 4 abgezweigt, über den kontinuierlich ein kleiner Teil des Feuchtgases F entnommen wird. In dem Probegasstrang 4 ist zunächst eine Einrichtung 10 geschaltet, mittels welcher der Masseanteil $X_w$ des in dem Feuchtgas F enthaltenen Wassers W in erfindungsgemäßer Weise bestimmt wird. Das Wasser W bildet somit im Sinne der vorstehenden Definition die "erste Komponente" des Feuchtgases F. Zwischen den übrigen Komponenten D des Feuchtgases F (insbesondere also CO, $CO_2$, $N_2$ und $H_2$) wird nachfolgend aus Gründen der Vereinfachung nicht weiter differenziert.

Der Einrichtung 10 ist innerhalb des Probegasstrangs 4 eine Gasanalyseeinheit 11 nachgeschaltet, bei der es sich beispielsweise um einen Gas-Chromatographen handelt. Alternativ hierzu kann die Gasanalyseeinheit 11 die Gaszusammensetzung aber auch durch Einsatz von resistiven oder kapazitiven Sensoren, durch Messung der Wärmeleitfähigkeit, der Wärmeleitkapazität und/oder des Brechungsindexes, durch Messung der Mikrowellen- und/oder Infrarot-Absorption, durch Messung der Raman-Streuung oder nach einem anderen gängigem Gasanalyseverfahren bestimmen.

Die Einrichtung 10 umfasst eingangsseitig einen Druckregler 12, insbesondere in Form eines gewöhnlichen Membranreglers, der durch Steuerung eines zugeordneten Drosselventils 13 den Druck des aus dem Hauptgasstrang 3 entnommenen Feuchtgases F auf einen, einem vorgegebenen Sollwert entsprechenden Druck p von beispielsweise etwa 2 bar mindert. Das Drosselventil 13 wird hierbei durch eine Heizung 14 erwärmt, um eine vorzeitige Kondensation von Wasser W sicher auszuschließen.

Kleinere Druckschwankungen auf schneller Zeitskala (Regelschwingungen) des Feuchtgases F, wie sie durch den Druckregler 12 regelmäßig verursacht werden, werden durch ein dem Drosselventil 13 nachgeschaltetes Dämpfungsglied in Form eines grobmaschigen Gasfilters 15 mit einer Porengröße von beispielsweise etwa 350 $\mu$m vergleichmäßigt.

Dem Gasfilter 15 ist ein erster (Coriolis-)Massenflussmesser 16 nachgeschaltet. Dieser Massenflussmesser 16 bestimmt unmittelbar den Massenfluss $m_1$ des innerhalb des Probegasstrangs 4 fließenden Feuchtgases F als erste Referenz-Durchflussrate $M_1$:

$$M_1 := m_1 \qquad\qquad\qquad\qquad\qquad\text{GLG 1}$$

Nach Durchlaufen des Massenflussmessers 16 wird das Feuchtgas F einem Kondensator 17 (oder Gaskühler) zugeführt, in dem das Feuchtgas F auf eine Kondensatortemperatur $T_K$ von beispielsweise 5 °C abgekühlt wird. Bei dieser Abkühlung kondensiert ein großer Teil des in dem Feuchtgas F enthaltenen Wassers W aus und sammelt sich als flüssiges Kondensat K in einer Kondensatfalle 18 des Kondensators 17. In geringerem Umfang werden durch den in dem Kondensator 17 ablaufenden Trennschritt neben dem Wasser W auch andere Komponenten D des Feuchtgases F abgetrennt, insbesondere $CO_2$, das sich in wässriger Lösung ebenfalls in der Kondensatfalle 18 sammelt.

Das sich in der Kondensatfalle 18 ansammelnde Kondensat K bildet somit ein Trenn-Fluid im Sinne der vorstehenden Definition. Das in dem Trennschritt getrocknete Synthesegas wird entlang des Probegasstrangs 4 aus dem Kondensator 17 abgezogen und der Gasanalyseeinheit 11 zugeführt. Dieses getrocknete Synthesegas, das ein "Rest-Fluid" im Sinne der vorstehenden Definition bildet, ist nachfolgend als Trockengas T bezeichnet.

Das Trockengas T durchläuft einen zweiten (Coriolis-)Massenflussmesser 19, der innerhalb des Probegasstrangs 4 dem Kondensator 17 und der Gasanalyseeinheit 11 zwischengeschaltet ist. Durch diesen Massenflussmesser 19 wird direkt der Massenfluss $m_2$ des Trockengases T als zweite Referenz-Durchflussrate $M_2$ gemessen:

$$M_2 := m_2 \qquad\qquad\qquad\qquad\qquad\text{GLG 2}$$

Durch einen der Kondensatfalle 18 zugeordneten Füllstandsregler 20 werden der Kondensatspiegel in der Kondensatfalle 18 - und hierüber auch das freie Gasvolumen in dem Kondensator 17- zumindest näherungsweise konstant gehalten. Der Füllstandsregler 20 steuert hierzu getaktet ein Abzugventil 21 an, über welches überschüssiges Kondensat K aus der Kondensatfalle 18 abgezogen und in einen Kondensatstrang 22 eingespeist werden kann.

Optional enthält die Einrichtung 10 im Kondensatstrang 22 einen weiteren (Coriolis-)Massenflussmesser 23, mittels welchem eine Durchflussrate $m_3$ des Kondensats K gemessen werden kann. In alternativen Ausführungsformen der Einrichtung 10 kann dieser Massenfluss $m_3$ anstelle des Massenflusses $m_1$ oder $m_2$ als Referenz-Durchflussrate herangezogen werden.

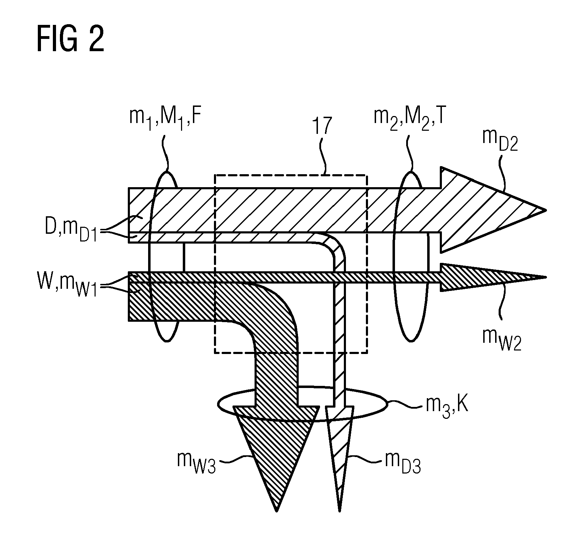

Die in dem Probegasstrang 4 und dem Kondensatstrang 18 fließenden Massenströme $m_1$, $m_2$ bzw. $m_3$ sind in FIG 2 nochmals schematisch verdeutlicht dargestellt. Aus dieser Darstellung ist insbesondere erkennbar, dass der Massenstrom $m_1$ des dem Kondensator 17 zugeführten Feuchtgases F sich aufteilen lässt in einen Wasser-Massenstrom $m_{W1}$ und einen Massenstrom $m_{D1}$ der übrigen Komponenten D. Ebenso lässt sich der Massenstrom $m_2$ des Trockengases T aufteilen in einen Wasser-Massenstrom $m_{W2}$ (der im Sinne der vorstehenden Definition den "Rest-Anteil" der ersten Komponente bildet) sowie in einen Massenstrom $m_{D2}$ der übrigen Komponenten D. Schließlich lässt sich auch der dem Kondensat K zugeordnete Massenstrom $m_3$ aufteilen in einen Wasser-Massenstrom $m_{W3}$ sowie in einen Massenstrom $m_{D3}$ der übrigen Komponenten D. Die letztgenannte Massenstrom $m_{D3}$ bildet im Sinne der vorstehenden Definition den "Fremd-Anteil" der übrigen Komponenten D in dem Trenn-Fluid.

Die Massenflussmesser 16 und 19 sowie - falls vorhanden - der Massenflussmesser 23 sind signaltechnisch mit einer Auswerteeinheit 24 der Einrichtung 10 verschaltet und führen dieser Auswerteeinheit 24 fortlaufend Messwerte der Referenz-Durchflussraten $M_1$ und $M_2$ sowie optional des Massenstroms $m_3$ zu.

Bei der Auswerteeinheit 24 handelt es sich vorzugsweise um einen Rechner, insbesondere einen PC mit einem darauf softwaretechnisch implementierten Auswerteprogramm 25. Durch dieses Auswerteprogramm 25 wird der verfahrensgemäß zu bestimmende Masseanteil $X_W$ des in dem Feuchtgas F enthaltenen Wassers W anhand der Referenz-Durchflussraten $M_1$ und $M_2$ bestimmt nach der Formel

$$x_W = \frac{1}{1+\beta} + \left(1 - \frac{1}{1+\beta} - \frac{1}{1+\alpha}\right)\frac{M_2}{M_1} \qquad \text{GLG 3}$$

In GLG 3 steht die Größe $\alpha = \alpha(T_K)$ für den Massendichteanteil von Wasser W im Trockengas T, die dem Sättigungsdampfdruck $p_s$ von Wasser bei der Kondensatortemperatur $T_K$ entspricht.

Die Größe $\beta = \beta(T_K)$ steht für die Gleichgewichtslöslichkeit von Kohlendioxid in Wasser W bei der Kondensatortemperatur $T_K$.

Der Massendichteanteil $\alpha$ wird vorzugsweise näherungsweise über die für ein ideales Gas geltende Zustandsgleichung ($p.V = v.R_m.T$) aus dem Sättigungsdampfdruck $p_S(T_K)$ abgeleitet:

$$\alpha(T_K) = \frac{\rho_W(T_K)}{\rho_T(T_K)} = \frac{v_W(T_K)\cdot M_W}{\rho_T(T_K)\cdot V} = \frac{p_S(T_K)\cdot M_W}{\rho_T(T_K)\cdot R_m\cdot T_K} \qquad \text{GLG 4}$$

In GLG 4 stehen die Größen

- $\rho_W = \rho_W(T_K)$     für die Massendichte des im Trockengas T enthaltenen Wassers W bei der Kondensator-temperatur $T_K$,
- $\rho_T = \rho_T(TK)$     für die Massendichte des Trockengases T bei der Kondensatortemperatur $T_K$,
- V     für das betrachtete Gasvolumen,
- $v_W = v_W(T_K)$     für die Stoffmenge (Molanzahl) der Wassermoleküle in dem Gasvolumen V bei der Kondensatortemperatur $T_K$,
- $M_w$     für die molare Masse von Wasser, und
- $R_m$     für die allgemeine Gaskonstante R = 8, 3144721 J·(mol·K)$^{-1}$.

Die Massendichte $\rho_T$ des Trockengases T wird durch die Gasanalyseeinheit 11 messtechnisch bestimmt. Anstelle der Zustandsgleichung für ideale Gase kann für die Ableitung des Massedichteanteils $\alpha$ aus dem Sättigungsdampfdruck $p_s(T_K)$ auch eine für reale Gase geltende Zustandgleichung (z.B. gemäß Peng-Robinson oder Redlich-Kwong-Soave) herangezogen werden.

Die temperaturabhängigen Werte für die Größen $\alpha$ und $\beta$ entnimmt das Auswerteprogramm 25 hinterlegten Kennlinien, wobei es einen messtechnisch bestimmten Wert für die Kondensatortemperatur $T_K$ zugrunde legt. Alternativ hierzu kann die Kondensatortemperatur $T_K$ im Rahmen des Auswerteprogramms 25 auch fest voreingestellt sein, so dass eine Messung dieser Temperatur entfallen kann.

Die GLG 3 ergibt sich rechnerisch aus dem Umstand, dass der

Massenstrom $m_{W2}$ (Restanteil) des im Trockengas T noch enthaltenen Wassers W über den Wert des Massendichteanteils $\alpha$ proportional zu dem Massenstrom $m_{D2}$ der übrigen Komponenten D in dem Trockengas T ist:

$$m_{W2} = \alpha(T_K) \cdot m_{D2} \qquad\qquad \text{GLG 5}$$

Weiterhin liegt GLG 3 die Annahme zugrunde, dass der im Kondensat K enthaltene Massenstrom $m_{D3}$ der übrigen Komponenten D über die Kohlendioxid-Löslichkeit $\beta$ proportional ist zu dem Massenstrom $m_{W3}$ des im Kondensat K enthaltenen Wassers W:

$$m_{D3} = \beta(T_K) \cdot m_{W3} \qquad\qquad \text{GLG 6}$$

Ferner liegt GLG 3 die Annahme zugrunde, dass sich die in FIG 2 dargestellten Massenströme zu Null aufheben:

$$m_1 - m_2 - m_3 = (m_{W1} + m_{D1}) - (m_{W2} + m_{D2}) - (m_{W3} + m_{D3}) = 0 \qquad \text{GLG 7}$$

Dass diese Annahme mit hoher Genauigkeit erfüllt ist, wird im Rahmen der Einrichtung 10 insbesondere durch den Druckregler 12 sowie durch den Füllstandsregler 20 gewährleistet.

In GLG 2 geht schließlich die Erkenntnis ein, dass der Massenstrom $m_{D3}$ der im Kondensator 17 als "Fremd-Anteil" mitabgetrennten weiteren Komponenten D fast ausschließlich aus Kohlendioxid besteht, zumal die übrigen Bestandteile CO, $N_2$ und $H_2$ des Feuchtgases F nur eine deutlich geringere Löslichkeit in Wasser haben.

Die GLG 2 und die darauf aufbauende GLG 3 können aber leicht auf Ausführungsformen der Erfindung erweitert werden, in denen in dem Fremd-Anteil, d.h. dem Massenstrom $m_{D3}$, außer $CO_2$ mehrere weitere Komponenten D in signifikaten Anteilen vorhanden sind. In diesem Fall ist die Größe $\beta$ als Summe von entsprechenden Löslichkeiten $\beta_i$ (i = 1,2,...,N) jeder zu berücksichtigenden Komponente zu berechnen:

$$\beta = \beta_1 + ... + \beta_N \qquad\qquad \text{GLG 8}$$

Entsprechend kann auch GLG 1 auf mehrere Komponenten erweitert werden:

$$\alpha = \alpha_1 + ... + \alpha_N \qquad\qquad \text{GLG 9}$$

Des Weiteren kann die erfindungsgemäße Einrichtung 10 - gegebenenfalls in abgewandelter Form - auch zur Bestimmung des Masseanteils einer ersten Komponente aus einem anderen Mehrkomponenten-Fluid herangezogen werden. Insbesondere kann mittels einer modifizierten Ausführung der Einrichtung 10 der Feststoffanteil in einem Flüssigkeits-Feststoff-Gemisch (z.B. einem Sand-Wasser-Gemisch) bestimmt werden. Für diese Anwendung umfasst die Einrichtung 10 insbesondere anstelle des Kondensators 17 einen Feststoffabscheider.

Die Erfindung beschränkt sich nicht auf die vorstehend beschriebenen Ausführungsbeispiele. Vielmehr können weitere Ausführungsformen der Erfindung von dem Fachmann aus der vorstehenden Beschreibung abgeleitet werden.

**Patentansprüche**

1.  Verfahren zur Bestimmung eines Masseanteils einer ersten Komponente (W) eines Mehrkomponenten-Fluids (F),

    - bei welchem die erste Komponente (W) in einem Trennschritt zumindest teilweise aus dem Mehrkomponenten-Fluid (F) abgetrennt wird,
    - bei welchem mindestens zwei Referenz-Durchflussraten ($M_1, M_2$) bestimmt werden, die gewählt sind aus

        -- -einer Durchflussrate ($m_1$) des dem Trennschritt zugeführten Mehrkomponenten-Fluids (F),
        -- einer Durchflussrate ($m_2$) eines aus der Abtrennung der ersten Komponente (W) resultierenden Rest-Fluids (T), und
        -- einer Durchflussrate ($m_3$) eines in dem Trennschritt anfallenden Trenn-Fluids (K), und

    - bei welchem aus den gewählten Referenz-Durchflussraten ($M_1, M_2$) der Masseanteil ($X_W$) der ersten Komponente (W) unter Berücksichtigung eines nicht abgetrennten Restanteils ($m_{W2}$) der ersten Komponente (W) in dem Rest-Fluid (T) bestimmt wird,
    **dadurch gekennzeichnet, dass**
    ein Fremdanteil ($m_{D3}$) einer in dem Trennschritt mitabgeteilten weiteren Komponente (D) des Mehrkomponenten-Fluids (F) berücksichtigt wird.

2.  Verfahren nach Anspruch 1,
    bei welchem die Referenz-Durchflussraten ($M_1, M_2$) durch direkte Messung der Massenflussrate ($m_1, m_2$) des Mehrkomponenten-Fluids (F), des Rest-Fluids (T) bzw. des Trenn-Fluids (K) bestimmt werden.

3.  Verfahren nach Anspruch 2,
    bei welchem die Bestimmung der Referenz-Durchflussraten ($M_1, M_2$) jeweils mittels eines Coriolis-Massenflussmessers (16,19) erfolgt.

4.  Verfahren nach einem der Ansprüche 1 bis 3,
    bei welchem der Druck (p) des dem Trennschritt zugeführten Mehrkomponenten-Fluids (F) auf einen vorgegebenen Sollwert geregelt wird.

5.  Verfahren nach einem der Ansprüche 1 bis 4,
    bei welchem regelungsbedingte Druckschwankungen des dem Trennschritt zugeführten Mehrkomponenten-Fluids (F) durch ein dem Druckregler (12) nachgeschaltetes Dämpfungsglied (15), insbesondere in Form eines Filters, reduziert werden.

6.  Verfahren nach einem der Ansprüche 1 bis 5,
    bei welchem als Mehrkomponenten-Fluid (F) ein Gasstrom herangezogen wird, und bei welchem in dem Trennschritt das Trenn-Fluid (K) durch Kondensation in einem Kondensator (17) abgetrennt wird.

7.  Verfahren nach Anspruch 6,
    wobei der nicht abgetrennte Restanteil ($m_{W2}$) der ersten Komponente (W) in dem Rest-Fluid (T) anhand des Sättigungsdampfdrucks ($p_s$) bei der Kondensatortemperatur ($T_K$) bestimmt wird.

8.  Verfahren nach Anspruch 6 oder 7,
    wobei der Fremdanteil ($m_{D3}$) der weiteren Komponente (D) in dem Trenn-Fluid (K) anhand der Löslichkeit ($\beta$) der weiteren Komponente (D) in der ersten Komponente (W) bei der Kondensatortemperatur ($T_K$) bestimmt wird.

9.  Verfahren nach den Ansprüchen 7 und 8,
    wobei der Masseanteil ($X_W$) der ersten Komponente (W) nach der Gleichung

$$x_W = \frac{1}{1+\beta} + \left(1 - \frac{1}{1+\beta} - \frac{1}{1+\alpha}\right)\frac{M_2}{M_1}$$

bestimmt wird, wobei

- $X_W$ der Masseanteil der ersten Komponente(W),
- $\alpha = \alpha(T_K)$ ein aus dem Sättigungsdampfdruck ($p_s$) bei der Kondensatortemperatur ($T_K$) abgeleiteter Massedichteanteil der ersten Komponente (W) im Restgas (T),
- $\beta = \beta(T_K)$ die Löslichkeit der weiteren Komponente (D) in der ersten Komponente (W) bei der Kondensator-temperatur ($T_K$),
- $M_1 := m_1$ der als erste Referenz-Durchflussrate gewählte Massenstrom ($m_1$) des dem Trennschritt zugeführten Mehrkomponenten-Fluids (F), und
- $M_2 := m_2$ der als zweite Referenz-Durchflussrate gewählte Massenstrom ($m_2$) des durch die Abtrennung der ersten Komponente (W) resultierenden Rest-Fluids (T)

sind.

10. Verfahren nach eine der Ansprüche 6 bis 9,
wobei das freie Gasvolumen in dem Kondensator (17) durch geregelten Abzug des Trenn-Fluids (K) aus dem Kondensator (17) konstant gehalten wird.

11. Einrichtung (11) zur Bestimmung eines Masseanteils einer ersten Komponente (W) eines Mehrkomponenten-Fluids (F) nach dem Verfahren nach einem der Ansprüche 1 bis 10,

- mit einer Trenneinheit (17) zur zumindest teilweisen Abtrennung der ersten Komponente (W) aus dem Mehrkomponenten-Fluid (F),
- mit einer ersten Durchflussraten-Messeinheit (16) zur Messung der ersten Referenz-Durchflussrate ($M_1$) und mit einer zweiten Durchflussraten-Messeinheit (19) zur Messung der zweiten Referenz-Durchflussrate ($M_2$), und
- mit einer Auswerteeinheit (24) , die dazu eingerichtet ist, aus den Referenz-Durchflussraten ($M_1, M_2$) den Masseanteil ($X_W$) der ersten Komponente (W) unter Berücksichtigung des nicht abgetrennten Restanteils ($m_{W2}$) der ersten Komponente (W) in dem Rest-Fluid (T) zu bestimmen,
**dadurch gekennzeichnet, dass**
die Auswerteeinheit (24) dazu eingerichtet ist, den Fremdanteil ($m_{D3}$) der in dem Trennschritt mitabgeteilten weiteren Komponente (D) des Mehrkomponenten-Fluids (F) zu berücksichtigen.

12. Einrichtung (11) nach Anspruch 11,
wobei der Trenneinheit (17) ein Druckregler (12) vorgeschaltet ist.

13. Einrichtung (11) Anspruch 12,
wobei dem Druckregler (12) und der Trenneinheit (17) ein Filter (15) zur Dämpfung regelungsbedingter Druckschwankungen zwischengeschaltet ist.

14. Einrichtung (11) nach einem der Ansprüche 11 bis 13, wobei die Trenneinheit als Kondensator (17) ausgebildet ist.

15. Einrichtung (11) nach einen der Ansprüche 11 bis 14, wobei dem Kondensator (17) ein Füllstandregler (20) zur Konstanthaltung des freien Gasvolumens durch geregelten Abzug des Trenn-Fluids (K) beigeordnet ist.

FIG 1

EP 2 562 541 A1

## FIG 2

Europäisches
Patentamt

European
Patent Office

Office européen
des brevets

**EUROPÄISCHER RECHERCHENBERICHT**

Nummer der Anmeldung

EP 11 17 8490

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | DE 44 33 451 A1 (TESTO GMBH & CO [DE]) 21. März 1996 (1996-03-21) * das ganze Dokument * ----- | 1,6,7, 11,14 | INV. G01N33/00 G01F1/74 |
| A | US 5 050 109 A (LADD MICHAEL M [US]) 17. September 1991 (1991-09-17) * Zusammenfassung; Abbildung 1 * * Spalte 2, Zeile 63 - Spalte 3, Zeile 28 * <br> * Spalte 5, Zeile 43 - Spalte 7, Zeile 7 * ----- | 1,4,6, 11,12 | |
| A | US 2003/136185 A1 (DUTTON ROBERT E [US] ET AL) 24. Juli 2003 (2003-07-24) * Absätze [0003], [0007], [0017] - [0020], [0055] - [0063], [0075], [0082] - [0107]; Abbildungen 1,2 * ----- | 1-4,11, 12 | |
| | | | **RECHERCHIERTE SACHGEBIETE (IPC)** <br> G01N <br> G01F |

Der vorliegende Recherchenbericht wurde für alle Patentansprüche erstellt

| Recherchenort | Abschlußdatum der Recherche | Prüfer |
|---|---|---|
| München | 11. Januar 2012 | Meyer, Fred |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..........................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 11 17 8490

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-01-2012

| Im Recherchenbericht angeführtes Patentdokument | Datum der Veröffentlichung | Mitglied(er) der Patentfamilie | Datum der Veröffentlichung |
|---|---|---|---|
| DE 4433451 A1 | 21-03-1996 | DE 4433451 A1<br>EP 0703441 A1<br>JP 8101111 A | 21-03-1996<br>27-03-1996<br>16-04-1996 |
| US 5050109 A | 17-09-1991 | KEINE | |
| US 2003136185 A1 | 24-07-2003 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4433451 A1